Europäisches Patentamt

European Patent Office

Office européen des brevets

(11) Numéro de publication: **0 270 459 B1**

# FASCICULE DE BREVET EUROPEEN

(45) Date de publication de fascicule du brevet: **21.10.92**  (51) Int. Cl.⁵: **C02F 3/28**, B65D 88/76

(21) Numéro de dépôt: **87402736.0**

(22) Date de dépôt: **02.12.87**

Le dossier contient des informations techniques présentées postérieurement au dépôt de la demande et ne figurant pas dans le présent fascicule.

(54) **Procédé et installation pour la production de biogaz.**

(30) Priorité: **02.12.86 FR 8616816**

(43) Date de publication de la demande:
**08.06.88 Bulletin 88/23**

(45) Mention de la délivrance du brevet:
**21.10.92 Bulletin 92/43**

(84) Etats contractants désignés:
**AT BE CH DE ES FR GB IT LI NL SE**

(56) Documents cités:
EP-A- 0 048 675    EP-A- 0 056 202
DE-A- 3 151 187    FR-A- 900 026
FR-A- 2 475 524    FR-A- 2 481 873

(73) Titulaire: **VALORGA PROCESS SA**
**5, Rue de Massacan**
**F- Z.I. VENDARGUES 34740(FR)**

(72) Inventeur: **Legratiet, Yves**
**Moulin de Naviteau 3 Chemin des Martinets**
**F-34170 Castelneau Le Lez(FR)**
Inventeur: **Kloppenburg, Jean**
**6 rue Brocherie**
**F-38000 Grenoble(FR)**
Inventeur: **Cayrol, François**
**10 rue des Pins**
**F-34000 Montpellier(FR)**

(74) Mandataire: **Beauchamps, Georges et al**
**Cabinet Z.Weinstein 20, avenue de Friedland**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

# Description

La présente invention intéresse le traitement biologique de matières ou substrats soumis à l'action de colonies de microorganismes, traitement global dans lequel interviennent au moins deux phases successives chacune correspondant à une réaction biochimique dominante.

L'invention intéresse plus spécialement un procédé, et une installation en vue de la mise en oeuvre de ce procédé, permettant la production dans des conditions améliorées de biogaz, obtenue notamment par fermentation bactérienne de matières organiques. Et c'est en rapport avec cette application que l'invention sera ci-après décrite, sans cependant être limitée à ce domaine.

En effet la présente invention est particulièrement adaptée à la mise en oeuvre d'un procédé permettant l'exploitation et la récupération de déchets d'origine urbaine, agricole ou éventuellement industriels, par fermentation en milieu anaérobie, cette fermentation, sous l'action de colonies de bactéries, s'accompagnant de la production d'un gaz comportant une proportion importante de méthane et dit "biogaz".

L'invention concerne plus spécialement l'exploitation de résidus se présentant au départ sous forme solide.

Il est intéressant de pouvoir traiter les matières soumises à la réaction de fermentation anaérobie, en continu, dans un même réacteur, ce qui permet de façon évidente de réduire les coûts d'investissement et d'exploitation en main d'oeuvre, énergie et entretien.

On distingue traditionnellement six types de procédés en continu, appliqués au traitement de liquides :
- infiniment mélangé, avec une agitation mécanique ou pneumatique de la cuve de fermentation ;
- contact anaérobie, avec un recyclage d'une partie des microorganismes sortants, et récupérés dans un décanteur interne, dans la cuve de fermentation pour augmenter la concentration bactérienne moyenne ;
- filtre anaérobie, avec la fixation des bactéries sur un garnissage inerte intérieur à la cuve ;
- lit fluidisé, avec le maintien en suspension d'un garnissage par un flux ascendant du liquide à traiter ;
- UASB, avec un décanteur interne à la cuve de fermentation piégeant des granulats de boues bactériennes ;
- piston, avec un front de progression de la matière de manière homogène. Une approche en est faite par le passage des effluents dans une succession de cuves de type infiniment mélangé.

La demande de brevet européen N° 0 048 675 décrit un appareil de traitement de déchets biochimiques, du type liquide. Cet appareil comprend une enceinte formant réacteur, munie de cloisons séparatrices verticales délimitant des volumes de gaz et de matière où ont lieu chacune des phases de la fermentation.

Cependant, s'il est relativement aisé de mettre en oeuvre des procédés continus à partir d'une matière première liquide, l'exploitation de procédé continu, dans le cas de déchets solides ou pâteux est beaucoup moins aisée.

On connaît ainsi des procédés dans lesquels les matières à traiter sont contenues dans des paniers se déplaçant dans un tunnel étant immergées en milieu liquide, l'entraînement étant obtenu par des systèmes mécaniques complexes tels que vis, chaîne ou racleur ; il est évident que ces installations sont difficiles à contrôler et représentent un investissement élevé et des risques de disfonctionnement mécaniques qui n'ont pas permis un développement industriel.

On connaît également un procédé de traitement d'une matière première contenant des teneurs élevées en matière sèche, les matières solides étant mises en suspension de façon à obtenir une masse pâteuse permettant un comportement visqueux, sensiblement voisin d'une masse semi-liquide, on peut ainsi exploiter et traiter des ordures ménagères d'origine urbaine, les matières premières étant ramenées dans ce cas à une granulométrie comprise entre 0 et 100 mm, sont légèrement diluées à un taux de matière sèche compris entre 30 à 35% d'extraits secs ; les matières introduites en force par un puits ou un système à piston dans un réacteur cylindrique où ils peuvent y subir un cycle de traitement avec un temps de rétention hydraulique (RTH) appropriée de l'ordre de 15 à à 25 jours.

La demande de brevet français N° 2 481 873, a pour objet une installation de fermentation pour la production continue de gaz méthane à partir de matières liquides ou non-liquides baignant dans un liquide. De plus, les dimensions et la capacité de chacun des volumes correspondants soit à la fermentation aérobie, soit à la fermentation anaérobie sont calculés avant le lancement de la fermentation, et aucun recyclage des matières, ni contrôle des paramètres biologiques n'est prévu au cours de la fermentation.

On connaît également un procédé de fermentation continu comportant la mise en place des substrats (tels que fumier) dans un silo vertical ; les matières sont introduites par le haut et soutirées à la base ce qui entraîne cependant des problèmes techniques ; ces procédés sont en outre limités en volume disponible en raison de la configuration verticale de l'installation ce qui limite l'application de ce procédé à de grandes installations.

Il est apparu cependant que si la mise en oeuvre d'un procédé continu présentait des avantages au niveau de l'exploitation, elle présentait l'inconvénient de maintenir dans une enceinte aux conditions uniques, des matières en début de dégradation et voisinant par conséquent avec des matières déjà en fin de dégradation et avancées dans le processus complexe de méthanisation par digestion bactérienne, de sorte que dans ce milieu hétérogène les conditions moyennes (température, composition, gaz produit, taux de recyclage, pH, et...) ne correspondent pas nécessairement aux conditions optimales de chaque phase caractérisée par une réaction biochimique dominante du processus global.

Cependant, cet avantage des procédés continus reste lui-même assez théorique car il supposerait que chaque charge soit elle-même d'une composition homogène de sorte que les différentes phases du traitement de méthanisation soient identiques pour chaque élément composant ; or par définition, si les charges notamment dans le cas de résidus d'origine urbaine, ont une composition moyenne sensiblement constante dans le temps, il reste que chaque charge alimentant le réacteur continu contient des éléments de compositions parfaitement hétérogènes, depuis des matières rapidement fermentescibles, jusqu'à des produits dont la réaction à l'action de digestion des bactéries sera au contraire beaucoup plus lente ; de sorte que l'on retrouve l'inconvénient précisé précédemment à savoir que même dans un réacteur continu, après quelques temps dans l'avancement du cycle, la charge contient des matières qui sont à un degré relativement avancé dans la dégradation tandis que d'autres composants sont restés très en retard.

Enfin, la demande de brevet français N° 2 475 524 propose un procédé de fermentation comprenant deux phases : une prédigestion produisant du gaz carbonique, ayant lieu dans une fosse de prédigestion et une phase de digestion produisant du méthane, ayant lieu dans un couloir de fermentation distinct de la fosse de prédigestion et alimenté par la matière provenant de cette dernière.

On sait par ailleurs que le processus biochimique défini comme la méthanisation de produits organiques résulte en réalité de la succession de quatre réactions biologiques complexes réalisées dans des communautés microbiennes d'espèces différentes agissant en symbiose.

Et on distingue traditionnellement quatre phases successives de dégradation sous l'action des populations bactériennes dominantes successives :

- l'hydrolyse : les biopolymères constitutifs de la matière organique (glucides, protéines et lipides) qui sont solubilisés en substances métabolisables par les microorganismes. Cette phase peut se dérouler en milieu aérobie ou anaérobie selon les substrats ;
- l'acidogénèse : les intermédiaires précédents sont métabolisés par des bactéries acidogènes en un mélange complexe riche d'une part en acides gras volatils et autres constituants à plus de deux atomes de carbone, et d'autre part en acétate, dioxyde de carbone et hydrogène ;
- l'acétogénèse : sous l'action d'un nouveau groupe bactérien, les acides organiques et autres constituants à plus de deux atomes de carbone sont transformés en dioxyde de carbone, hydrogène et acide acétique ; on regroupe parfois les phases d'acidogénèse et d'acétogénèse ;
- la méthanogénèse : au cours de cette phase ultime sous l'action d'un dernier groupe de micro-organismes, l'acétate est transformé en biogaz, tandis que le dioxyde de carbone est réduit par l'hydrogène.

Il est reconnu que la vitesse de l'hydrolyse est en elle même plus faible que celle de la méthanogénèse, elle même plus faible que celle de l'acidonégèse et que biologiquement la vitesse de réaction est directement liée à la vitesse de reproduction des bactéries.

Il apparaît ainsi souhaitable, c'est là un premier objet de l'invention, de disposer d'un procédé permettant d'allier les avantages technologiques et d'économie à l'investissement et en cours d'exploitation des procédés continus, à une adaptation constante des paramètres de la conduite du traitement aux phases successives du processus, chaque phase correspondant à une réaction dominante spécifique devant par conséquent être conduite dans des conditions appropriées et adaptées.

Un autre objet de l'invention est la réalisation d'installations qui postérieurement à la conception et la réalisation initiale, présenteront des possibilités d'adaptation et une flexibilité leur permettent de répondre à tout changement quantitatif ou qualitatif des matières à traiter. Il est en effet très difficile de connaître la composition exacte, pour une ville, une région, ou même pour un pays, des rejets ou résidus appelés à être traités. De plus, les pourcentages des composants évoluent dans le temps avec les productions et les habitudes de consommation.

De sorte que le procédé de l'invention permettra d'optimiser le processus de méthanisation, au long duquel les diverses réactions dominantes sont successivement conduites dans un fermenteur unique mais dissocié virtuellement en zones de travail différenciées, chacune affectée à une réaction dominante.

A cet effet, l'invention concerne un procédé de traitement en continu de matières premières organiques à l'état solide ou pâteux, par fermentation sous l'action de colonies de microorganismes, et dans lequel interviennent au moins deux phases successives, chacune correspondant à une réaction biochimique dominante, du type dans lequel les matières à traiter sont introduites dans au moins un réacteur où elles subissent l'action des microorganismes, tels que des colonies de bactéries provoquant une fermentation anaérobie en vue de la production de biogaz, la matière étant appelée à transiter en continu entre une entrée principale et une sortie situées aux deux extrémités du réacteur, les matières transitant selon un temps de rétention hydraulique correspondant au temps de séjour nécessaire pour le traitement utile moyen des matières comprises dans l'alimentation, le procédé étant caractérisé en ce qu'il consiste à faire circuler la matière en continu dans le réacteur, longitudinalement et suivant un parcours non rectiligne, concave et notamment courbe ; à au moins prélever un flux de matière au moins en une partie convenable du cheminement pour la réintroduire en amont afin de réaliser un recyclage au moins partiel des matières traitées spécifiques à une zone de réaction dominante, le point d'introduction et le point de prélèvement des matières recyclées se situant respectivement sensiblement au point de sortie et au point d'entrée d'un volume virtuel définissant ladite zone au sein du réacteur ; à moduler le taux de recyclage au sein de ladite zone afin d'adapter le temps de séjour moyen des matières dans cette zone au temps de reproduction des colonies de microorganismes spécifiques de cette zone ; et à contrôler des conditions spécifiques à une réaction dominante au sein de chacune des zones successives en procédant régulièrement à la prise de mesures physiques et/ou chimiques et/ou biologiques propres à permettre la détermination de l'état de la matière et les conditions de déroulement de la réaction dominante au sein de chaque zone.

Dans le cadre de la mise en oeuvre du procédé, le fait de prévoir la modulation du taux de recyclage au sein d'une zone (les matières étant prélevées à la sortie de ladite zone et réintroduites en amont à l'entrée de cette zone), de façon à adapter le temps de séjour moyen des matières dans cette zone au temps de reproduction des colonies de microorganismes (biomasse) spécifique de cette zone, permet ainsi d'éviter le lessivage.

Selon une autre modalité de mise en oeuvre, on injecte dans le milieu en cours de transit, et de façon spécifique à chacune des zones, des agents notamment gazeux, tels que du méthane ou du dioxyde de carbone, propres à agir chimiquement sur l'équilibre du milieu, en fonction de la réaction dominante au sein de chaque zone.

Et plus spécialement, on prévoit de délimiter les atmosphères gazeuses régnant au-dessus de chaque zone en mettant en place une paroi supérieure transversale, aux endroits correspondant à la sortie d'une zone de réaction dominante et à l'entrée d'une zone ultérieure correspondant à une autre réaction dominante, les atmosphères gazeuses situées au-dessus de chaque zone étant ainsi isolées.

Plus particulièrement dans le cadre de la mise en oeuvre de l'invention pour la production de biogaz par fermentation bactérienne de résidus, on définit au sein du cheminement de faible section et de grande longueur correspondant au parcours des matières traitées, quatre zones successives correspondant chacune à une réaction dominante à savoir :

a) une première zone affectée à une réaction d'hydrolyse et située entre l'entrée principale et une première entrée secondaire.

b) une seconde zone en aval le long du cheminement des matières traitées, et affectée à une réaction d'acidogénèse, située entre la première entrée secondaire et une seconde entrée secondaire.

c) une troisième zone située à son tour en aval le long du cheminement des matières traitées, et affectée à une réaction d'acétogénèse, cette troisième zone étant située entre la seconde entrée secondaire et une troisième entrée secondaire.

d) une quatrième zone située en aval et correspondant à une réaction de méthanogénèse, ladite zone étant située entre la troisième entrée secondaire et la sortie des matières sous forme d'effluents,

Et on contrôle les conditions d'alimentation, de recyclage, de température, de réinjection de biogaz, au sein de chaque zone, en fonction de l'évolution de la réaction dominante au sein de cette zone.

On peut plus particulièrement introduire directement dans une zone déterminée, en mélange au sein de la masse des matières en traitement, des métabolites intermédiaires propres à la flore bactérienne spécifique de la réaction dominante se déroulant dans cette zone.

Et plus particulièrement, lesdits métabolites sont sous forme solide et sont choisis dans la famille comportant :

a) les produits riches en glucose introduits dans la zone affectée à l'acidogénèse ;

b) les produits riches en acétate introduits dans la zone de méthanogénèse.

L'invention concerne encore une installation en vue de la mise en oeuvre du procédé telle que définie précédemment.

A cet effet, l'invention concerne une installation pour le traitement de matières organiques, à l'état solide ou pâteux et subissant l'action de microorganismes tels que des colonies bactériennes notamment pour la production de biogaz, l'installation étant du type constitué d'une enceinte close pourvue d'une entrée principale des matières à traiter et d'une sortie des matières digérées sous forme d'effluents, caractérisée en ce que l'enceinte de section constante faible par rapport à son volume et suivant en plan un parcours en forme de segment de couronne, est reliée par une conduite d'entrée, une conduite de sortie et des moyens de prélèvement et d'introduction de matières ou de biogaz à une partie centrale dégagée située au centre dudit segment de couronne, constituant une aire technique comprenant des moyens de contrôle et de conduite du traitement dans l'enceinte en ses divers points.

Et plus spécialement, l'installation comporte le long de son parcours lesdits moyens de prélèvement de matière depuis le flux de matière en transit au sein de l'installation et lesdits moyens de réintroduction de matière vers le flux desdites matières en transit, ces moyens étant situés sur la paroi concave de l'enceinte de l'installation.

De préférence, on prévoit également que l'enceinte comporte sur sa partie supérieure, et venue de sa paroi formant couverture, des cloisons transversales sensiblement verticales et plongeant dans la matière en cours de réaction, ces cloisons étant aptes à diviser l'espace libre au-dessus de la matière pâteuse en transit dans le réacteur, en définissant des volumes gazeux indépendant les uns des autres, chaque volume étant relié à une unité centrale apte à recueillir séparément les prélèvements effectués dans lesdites phases gazeuses correspondant à chacun des volumes et d'une composition particulière.

D'autres caractéristiques et avantages de l'invention ressortiront de la description qui suit qui est donnée en rapport avec une forme de réalisation particulière présentée à titre d'exemple non limitatif et en se référant aux dessins annexés.

La figure 1 représente une vue en coupe transversale du réservoir selon l'invention ;

La figure 2 représente une vue en perspective d'une unité de traitement utilisant un réservoir selon l'invention ;

La figure 3 représente une vue en plan de l'installation de la figure 2 ;

La figure 4 représente une vue en perspective d'un élément unitaire de couverture.

Les figures 5a et 5b montrent une vue en plan d'une variante de réalisation convernant un réacteur de méthanisation comportant des zones virtuelles de travail ; la figure 5a révèle les circuits matière et la figure 5b les circuits gazeux.

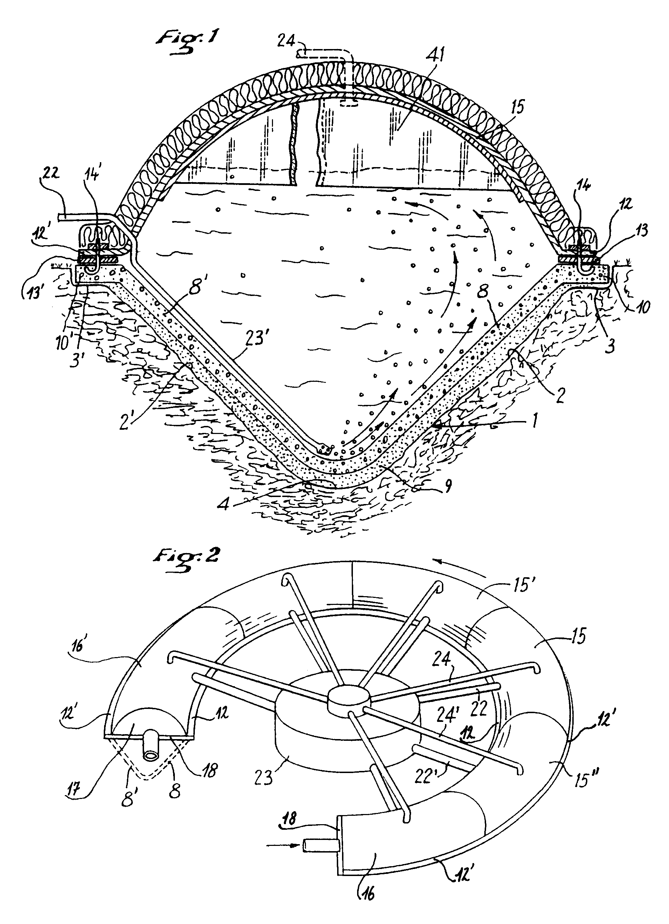

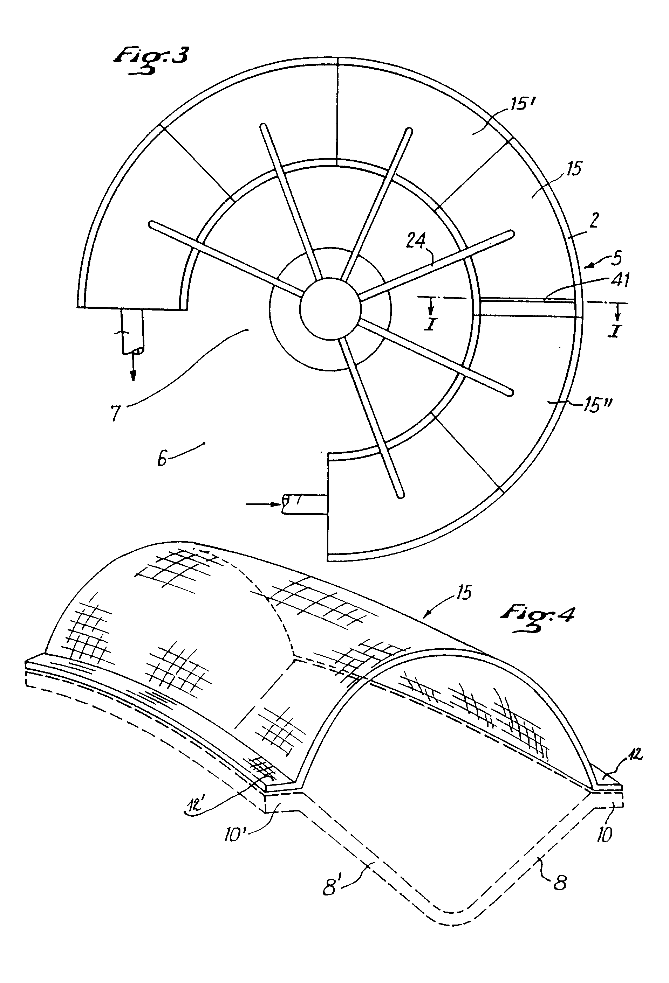

Selon l'ensemble des figures et notamment selon figure 1 qui représente une vue en coupe transversale selon la ligne I-I de la figure 3, on voit que le réservoir est semi-enterré et il est constitué à sa base d'une tranchée creusée à même le sol notamment par des moyens mécaniques tels qu'une pelleteuse.

La tranchée 1 est de forme évasée vers le sommet et selon les figures elle présente une section en forme de V comportant ainsi deux parois respectivement 2 et 2′ inclinées depuis le bord supérieur correspondant 3-3′ jusqu'au fond de la tranchée 4.

La tranchée, ainsi qu'on le voit sur la figure 2 et sur la figure 3, épouse un contour curviforme et de préférence, elle suit un parcours circulaire interrompu et elle est ainsi constituée en forme de segment de cercle 5 interrompu par la zone 6 permettant ainsi un accès depuis l'extérieur jusqu'au centre 7 de la couronne.

La tranchée étant ainsi effectuée et définissant un fond de forme constitué des deux parois inclinées 2,2′, on met en place un surfaçage ou paroi-revêtement disposé contre et parallèlement aux parois 2,2′.

Dans le cas illustré aux figures, on a mis en place un revêtement constitué d'une paroi en béton 8 reposant, de façon connue, sur un lit de grave 9.

Le revêtement de béton forme une chape épousant le fond à section en V de la tranchée et il est muni d'un radier d'armature épousant la conformation circulaire des parois 2,2′.

Facultativement, le béton pourra être coulé après mise en place de coffrages coulissants déplacés au fur et à mesure de l'avancement des travaux.

La tranchée pourra être revêtue de tout autre matériau de surfaçage assurant l'étanchéité des parois notamment des éléments préfabriqués en résine armée ou en acier chaudronné ou même éventuellement des films renforcés d'un matériau étanche souple (films plastique ou de caoutchouc armé).

On voit sur la figure 1 qu'en partie supérieure, les parois en béton 8 se prolongent par un massif 10,10′ situé à l'aplomb des bords supérieurs 3,3′ de la tranchée.

Ces massifs vont constituer les points d'appui formant culée pour la réception des éléments de couverture décrits ci-après.

Comme on le voit sur la figure 1, la tranchée ouverte est refermée à sa partie supérieure par un élément de fermeture et de recouvrement dont on a donné à la figure 4 une vue en perspective.

Cet élément peut être avantageusement réalisé en résine armée et il est dimensionné de façon à épouser en plan la courbure de la tranchée tandis que ses bords plans respectivement 12 et 12′

viennent se positionner au droit de la culée 10 ou 10' avec interposition de moyens d'étanchéité formés de matière spongieuse ou alvéolaire imprégnée de bitume 13,13'.

Une série de tiges filetées de préférence recourbée 14,14' noyée dans la culée 10 déborde en partie supérieure pour traverser le rebord 12,12' et assurer, au moyen d'un boulonnage, la fixation de ce bord sur la culée porteuse et on comprend que chaque élément de couverture 15 formant une voûte est ainsi autoporteur et peut être rapidement mis en place et solidarisé sur les bords formant culée 10,10' des parois inférieures 8,8' de la tranchée.

Les éléments 15 se juxtaposent les uns les autres de façon à former une couronne ou un segement de couronne qui se superpose exactement au segment de couronne 5 constitué par la tranchée.

Les éléments courants de couverture 15,15',15'' comportent, sur leurs bords adjacents venant en regard, des moyens d'engagement notamment sous forme de rainures coopérat entre elles avec interposition de moyens d'étanchéité (tels que des bandes de matériau compressible et alvéolaire imprégné de bitume) de sorte que l'étanchéité d'un élément 15 à l'autre est ainsi assurée.

Pareillement depuis l'unité centrale 23, peuvent rayonner les canalisations supérieures 24,24' qui recueillent le gaz produit à la partie du réservoir pour le centraliser et éventuellement le réintroduire dans le circuit de recirculation par les canalisations 22,22' après mise sous pression depuis le poste central 23.

Un avantage remarquable de l'invention est la grade souplesse dans la capacité du réacteur qui n'est pas figée définitivement.

Il est ainsi possible de réaliser un réservoir par exemple en quart ou en demi-cercle qui sera ensuite prolongé par l'adjonction d'un nouveau quart de cercle en réalisant ainsi une augmentation de la capacité tout en conservant l'unité et l'homogénéité de l'ensemble ; on comprend qu'il est facile lors de la réalisation des revêtements en béton armé 8 et 8' de laisser des fers d'attente débordant au-delà de la section transversale de fermeture, de sorte que, après destruction de cette section transversale, il est facile de prolonger la tranchée et de prolonger ensuite les parois 8 et 8' pour réaliser une nouvelle section de couronne réalisant l'extension du réservoir initial.

Le réservoir pourra ainsi, tout en assurant une grande capacité, être peu nuisible pour l'environnement et assumer ses fonctions de stockage selon un volume important sans cependant excéder une hauteur anormale.

On obtient grâce au dispositif de l'invention la possiblité de disposer au centre de la couronne (partiellement fermée) les installations centrales qui peuvent ainsi desservir l'ensemble du réacteur dont tous les points sont équidistants de la zone centrale.

Dans le cas où notamment il doit être apporté au produit en cours de réaction des éléments (réactif ou fluide de régulation thermique), on réduit considérablement les longueurs de canalisation.

Dans le cas où l'on utilise le réservoir en réacteur de méthanisation pour la fermentation et digestion bactérienne d'ordures ménagères, on peut notamment bénéficier, en utilisant le réservoir de l'invention, d'un abaissement de la consommation énergétique notamment à l'occasion de la mise en action des pompes d'injection de matière en raison de la faible hauteur de mise en charge due à la faible hauteur du digesteur.

Sur les éléments de couverture situés aux extrémités là où le segment de couronne formé par la tranchée est interrompu, on prévoit une obturation transversale.

Comme on le voit sur la figure 2, les éléments de couverture d'extrémité respectivement 16,16' comportent ainsi une paroi transversale 17 comportant elle-même un rebord 18 reposant et solidarisé sur une culée transversale prolongeant vers le haut une paroi transversale (non représentée sur les dessins) refermant les deux parois latérales 8 et 8' de la tranchée au niveau des deux extrémités.

Selon la figure 2, on voit ainsi que, depuis une source centrale contenant les pompes et compresseurs appropriés, peuvent rayonner les canalisations 22,22' (visibles à la figure 1) et aboutissant à des rampes ou buses de réinjection 23' plongeant au sein du réservoir et permettant ainsi la réinjection sous pression dans la masse visqueuse en cours de traitement de gaz riches en méthane, produisant ainsi les effets voulus dans le cadre du procédé ici concerné à savoir un accroissement du rendement en production méthanière.

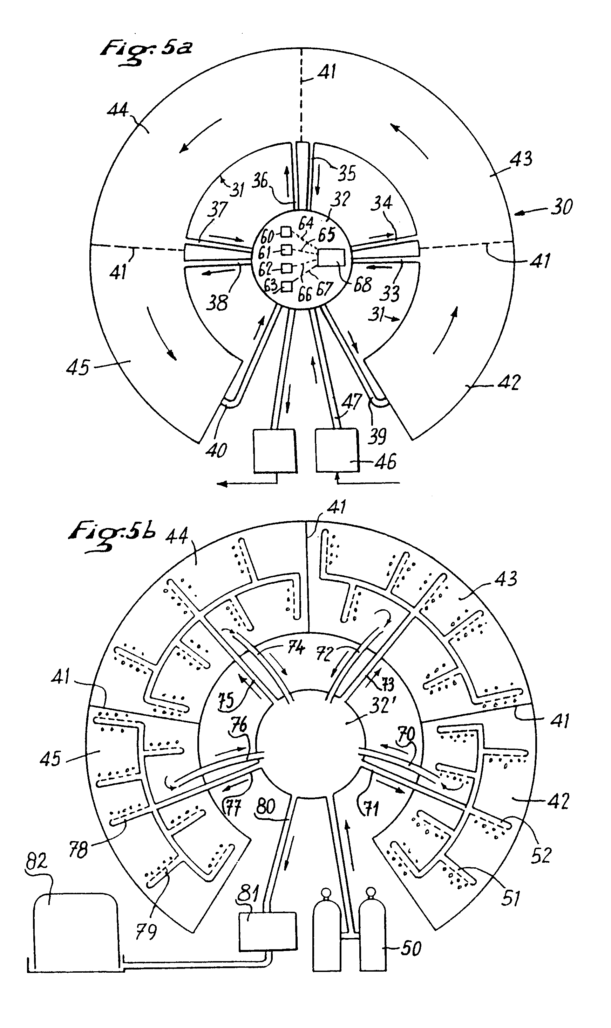

Les figures 5a et 5b donnent plus particulièrement un exemple de réalisation d'une installation prévue pour le traitement de résidus ou notamment d'ordures ménagères dans le cadre d'un processus de méthanisation conformément au procédé de l'invention.

La figure 5a montre le circuit des matières solides ou pâteuses traitées.

La figure 5b montre la circulation des phases gazeuses.

On voit que dans le cadre de cet exemple de mise en oeuvre on utilise un réacteur définissant une enceinte unique et telle que précédemment décrit en forme générale de segment de couronne.

Le réacteur 30 est ici relié par sa face concave 31 à une aire centrale 32, par une pluralité de conduites ou canalisations 33,34,35,36,37,38.

Ces canalisations constituent les moyens de transfert, d'introduction et de recyclage des matières en cours de transit au sein du réacteur 30.

On précisera que l'on a représenté des canalisations jumelées par exemple 33 et 34 chacune affectée à un sens de circulation, ceci pour la clarté et l'intelligence des dessins ; mais il sera plus simple de prévoir une seule canalisation qui suivant le sens d'utilisation servirait à la fois au retour des matières recyclées depuis le réacteur 30 vers l'aire technique 32 et inversement.

Selon cette forme de réalisation le réacteur réalise une enceinte unique parcourue de façon continue par les matières alimentées depuis la conduite 39, jusqu'à la sortie 40 par laquelle les matières traitées c'est-à-dire le digestat revient vers l'aire technique 32.

On a prévu cependant dans le cadre de cette réalisation que la partie supérieure du volume du réacteur comporte une pluralité de parois transversales telles qu'elles sont d'ailleurs représentées à la figure 1 et aux figures 5a et 5b sous la référence 41.

Ces parois transversales sont positionnées de façon à définir dans la partie supérieure de l'enceinte (affectée à la réception des atmosphères gazeuses, 4 zones qui vont correspondre à 4 zones de travail chacune affectée à une réaction dominante.

Pour la simplicité on a prévu 3 parois transversales 41 disposées de façon équidistante et définissant ainsi les zones 42,43,44 et 45 de volume sensible égal.

Mais il est évident que le positionnement des parois transversales 41 pourra être adjusté en fonction des nécessités c'est-à-dire en fonction du volume de la zone correspondant à une réaction déterminée ; chaque zone pourra ainsi recevoir au sein du volume général de l'enceinte du réacteur une longueur appropriée qui sera simplement définie par le positionnement de la paroi transversale 41.

Il est clair que cette paroi peut être aisément retirée et déplacée le long de la couronne si il apparaît nécessaire, en fonction de l'évolution ultérieure de l'alimentation, d'affecter un volume plus important à une zone de travail particulière.

On voit ainsi qu'il est possible d'obtenir une structure particulièrement souple et flexible puisque tout en conservant le réacteur intact, il est très simple de déplacer les limites virtuelles des zones de travail successives qui vont être ci-après décrites.

L'aire technique 32 comporte une pluralité de moyens permettant de desservir l'installation à savoir :

- des organes d'introduction, et de malaxage des matières à traiter entrant notamment depuis le stockage amont 46 et arrivant dans la zone centrale 32 par la conduite 47.

L'aire 32 comporte encore des moyens de mise en circulation, par exemple des pompes, aptes à assurer le mouvement des matières traitées et qui sont amenées à un état pâteux comme on le précisera ultérieurement.

Ainsi les matières entrantes et qui arrivent par la conduite ou le tapis 47, sont ramenées à une granulométrie appropriée et elles sont mises dans un état pâteux et fluide, permettant une circulation rhéologique des produits par mélange avec les jus provenant du digestat et évacué vers l'aire centrale en 40.

Eventuellement les matières entrantes peuvent déjà subir une élévation thermique pour les amener à un état physique propre à subir la première phase du processus global de méthanisation à savoir l'hydrolyse qui se situe dans la zone 42.

On comprend que c'est grâce à l'utilisation d'un parcours allongé et de faible section, et à l'état pâteux de la matière, que l'on peut ainsi obtenir des conditions locales différenciées dans chaque zone et adaptées à la réaction dominante de la zone correspondante, et sans interférer avec les zones voisines.

On peut ainsi prévoir dans la première zone 42 affectée à l'hydrolyse des conditions par exemple de température et de composition, ou encore de pH, contrôle de viscosité, etc... adaptés au développement de la réaction d'hydrolyse.

Notamment et en considérant la figure 5b on voit qu'il est possible d'initier le processus de méthanisation par une phase aérobie par exemple au sein de la zone 42 en injectant depuis une réserve de gaz neuf stockée 50, de l'oxygène voire de l'air arrivant aux rampes d'insufflation 51,52 qui tapissent la paroi inférieure du réacteur. L'atmosphère gazeuse éventuellement riche en oxygène qui peut être créée dans la zone 42 ne risque cependant pas de contaminer les zones ultérieures grâce aux bavettes ou parois transversales 41 qui isolent les atmosphères gazeuses de chaque zone les unes des autres.

De sorte que le gaz recueilli depuis la zone 42 par une canalisation supérieure peut être récupéré dans l'aire technique 32' (affectée au traitement des circuits gazeux) et y subir les opérations de séparation ou compression utiles (voir figure 5b).

La matière entrant par la canalisation 39, se trouve progressivement poussée vers l'aval au fur et à mesure de l'introduction de nouvelles matières subséquentes.

Au sein de la zone 42, les matières transitent lentement selon un temps de rétension hydraulique (TRH) approprié ; et au fur et à mesure de cet avancement les colonies de bactéries, correspondant spécifiquement à la réaction dominante dans la zone (hydrolyse) se développent et prospèrent.

Arrivé à proximité de la bavette transversale 41, les matières sont donc riches en colonies de bactéries correspondant à l'opération d'hydrolyse.

Et selon l'invention on procède alors à un prélèvement, par la canalisation 33, d'une partie des matières arrivées au terme de la phase d'hydrolyse, qui sont recyclées par l'aire technique 32 vers la canalisation entrante 39 où ces matières se mélangent avec la matière fraîche entrante pour apporter le levain et l'ensemencement en colonies de bactéries correspondant précisément à l'opération spécifique auquelle les matières entrantes vont être immédiatement soumises.

Pareillement on a prévu au niveau de l'aire technique, une pluralité de sondes de la matière et ces sondes sont schématisées en 60,61,62,63 et elles sont reliées par les circuits 64,65,66,67 à des moyens de contrôle 68 centralisant ainsi les informations permettant de suivre à travers les divers paramètres choisis l'évolution des réactions au sein des zones successives.

Ce dernier reçoit ainsi la mesure des températures tout au long du parcours ainsi que la mesure des viscosités, de l'acidité du milieu, ainsi que les mesures correspondant à la composition des gaz soutirés depuis les atmosphères gazeuses situées au sein de chaque zone et le débit de ces gaz, qui peut être mesuré au niveau des canalisations de retour gazeux respectivement 70,72,74,76 (figure 5b).

Il est ainsi possible à partir de l'aire technique, de connaître instantanément le degré d'aboutissement et de complétude des réactions des matières arrivées en fin d'une zone ou en cours de transit au sein de la zone et ceci permet d'influer sur les conditions prédominant au sein de cette zone de façon à accélérer, ralentir ou corriger de façon optimale le déroulement de la réaction.

A cet effet divers paramètres peuvent être mis en oeuvre - tels que le taux de recirculation des matières, depuis la canalisation de retour 33 (en considérant la zone 42).

Il est encore possible d'influer sur le processus en agissant sur les circuits gazeux comme on le voit sur la figure 5b et en agissant par conséquent sur la composition, le débit des gaz qui sont réinjectes dans la zone correspondante par les rampes inférieures 51,52.

Le transit des matières se poursuit régulièrement, n'étant handicapé par aucun obstacle au niveau de la phase solide ou pâteuse (seule la phase gazeuse est isolée par les cloisons transversales 41).

La matière hydrolysée entre donc dans la zone 43 où les paramètres contrôlés (température, pH, viscosité, etc... sont alors ajustés de façon à créer les conditions optimales de développement de la flore microbienne spécifique à la réaction d'acidogénèse.

Et comme on l'a indiqué précédemment, la matière entrant dans la zone 43 et provenant de la zone d'hydrolyse 42, reçoit l'apport, par la canalisation 34, d'une partie des matières recyclées depuis la canalisation 35, de sorte que les matières traitées dans le cours de la zone 43 reçoivent dès le départ un levain spécifique de la réaction et de la flore microbienne correspondant à cette réaction.

Les matières quittant la zone d'acidogénèse 43 vont donc entrer dans la zone d'acétogénèse 44 et comme précédemment décrit, elles recevront par la canalisation d'entrée 36 une partie recyclée des matières quittant la zone d'acétogénèse en introduisant ainsi à l'entrée de la zone d'acétogénèse le levain et l'ensemencement microbien spécifique et nécessaire à la réaction d'acétogénèse prévue dans cette zone.

Enfin le même phénomène se produit lorsque les matières riches en acétate quittant la zone d'acétogénèse entrent dans la zone 45 de méthanisation ; elles y reçoivent alors l'apport, par la canalisation 38, des matières recyclées depuis la sortie 40 et qui permettent l'introduction dès le début de la phase de méthanogénèse des bactéries spécifiques de cette réaction qui pourront donc se développer, les conditions de température, pH, etc..., précédemment évoquées, étant ajustées et contrôlées dans cette zone en fonction des paramètres souhaitables.

On peut également introduire à l'entrée de chaque zone des éléments intervenant dans les réactions soit à titre de métabolites, soit à titre d'agents chimiques créant les conditions propices au développpment des réactions dominantes de chaque zone.

A titre d'exemples les matières riches en glucose seront introduites dès la phase d'hydrolyse tandis que les matières riches en acétate seront introduites à l'entrée de la zone de méthanogénèse.

Il en est de même des flux gazeux ; on a vu que la zone 42 pouvait avantageusement recevoir un apport initial d'oxygène en vue d'un démarrage de la réaction en phase aérobie.

On pourra avantageusement récupérer le biogaz produit, spécifique à chaque zone, et après analyse de la composition, le réinjecter dans la même ou une autre zone, soit en aval, soit en amont où il pénètre par injection au niveau des rampes inférieures, dans la masse pâteuse afin d'y créer des conditions chimiques ou biochimiques propices à la réaction dominante de la zone.

On a ainsi prévu d'injecter par exemple du méthane à l'état pur, ou sous forme de biogaz produit dans le site, dans telle ou telle zone afin d'y ajuster le pH et de le corriger.

On prévoit également de diriger depuis la source de gaz neuf 50, de l'hydrogène arrivant par les rampes inférieures 78,79 dans la matière en cours de transit dans la zone 45 de méthanogénèse, l'hydrogène insufflé sous pression participant alors à la réduction du $CO_2$ notamment dissout pour la production de méthane.

On n'a pas reproduit sur la figure 5b, au niveau de l'aire technique 32' affectée à la régulation des circulations des gaz, les détails comportant notamment les registres, vannes, compresseurs, etc... ; on voit que le gaz finalement produit peut être évacué par la canalisation 80 vers une phase de purification 81 et stocké dans le gazomètre 82. On notera que le circuit des rampes de réinjection à la base des matières en transit, a été représenté étant divisée en quatre zones ; ceci pour la clarté des dessions ; dans la réalité les rampes pourront être reliées de façon directe à la zone ou aire technique centrale 32′, et il suffira de commander par des moyens de motorisation, la connexion des rampes à tel ou tel circuit de façon à prévoir l'injection d'un gaz approprié dans la zone définie qui ne sera donc pas géographiquement cristallisée.

**Revendications**

1. Procédé de traitement en continu de matières premières organiques à l'état solide ou pâteux, par fermentation sous l'action de colonies de microorganismes, et dans lequel interviennent au moins deux phases successives, chacune correspondant à une réaction biochimique dominante, du type dans lequel les matières à traiter sont introduites dans au moins un réacteur où elles subissent l'action des microorganismes, tels que des colonies de bactéries provoquant une fermentation anaérobie en vue de la production de biogaz, la matière étant appelée à transiter en continu entre une entrée principale et une sortie situées aux deux extrémités du réacteur, les matières transitant selon un temps de rétention hydraulique correspondant au temps de séjour nécessaire pour le traitement utile moyen des matières comprises dans l'alimentation, le procédé étant caractérisé en ce qu'il consiste :
à faire circuler la matière en continu dans le réacteur, longitudinalement et suivant un parcours non rectiligne, concave et notamment courbe ;
à au moins prélever un flux de matière au moins en une partie convenable du cheminement pour la réintroduire en amont afin de réaliser un recyclage au moins partiel des matières traitées spécifiques à une zone de réaction dominante, le point d'introduction et le point de prélèvement des matières recyclées se situant respectivement sensiblement au point d'entrée et au point de sortie d'un volume virtuel définissant ladite zone au sein du réacteur ;
à moduler le taux de recyclage au sein de ladite zone afin d'adapter le temps de séjour moyen des matières dans cette zone au temps de reproduction des colonies de microorganismes spécifiques de cette zone ; et
à contrôler des conditions spécifiques à une réaction dominante au sein de chacune des zones successives en procédant régulièrement à la prise de mesures physiques et/ou chimiques et/ou biologiques propres à permettre la détermination de l'état de la matière et les conditions de déroulement de la réaction dominante au sein de chaque zone.

2. Procédé selon la revendication 1, caractérisé en ce que le recyclage des matières entre la sortie d'une zone de travail, spécifique à une réaction dominante, et l'entrée de cette zone, est ajusté pour permettre l'ensemencement des matières nouvelles entrant dans la zone, en colonies de microorganismes spécifiques de la réaction dominante au sein de cette zone.

3. Procédé selon l'une des revendications 1 à 2, caractérisé en ce qu'il consiste de plus à délimiter les atmosphères gazeuses régnant au-dessus de chaque zone en mettant en place un moyen de séparation tel qu'une paroi supérieure transversale, aux endroits correspondant à la sortie d'une zone de réaction dominante et à l'entrée d'une zone ultérieure correspondant à une autre réaction dominante, les atmosphères gazeuses situées au-dessus de chaque zone étant ainsi isolées l'une de l'autre.

4. Procédé selon l'une des revendications 1 à 3, caractérisé en ce qu'il consiste de plus à recycler du biogaz soutiré de l'atmosphère gazeuse d'une zone dans la matière en cours de traitement au sein d'une zone antérieure ou postérieure, le biogaz intervenant à titre d'agent chimique ou biochimique dans le développement de la réaction dominante.

5. Procédé selon l'une des revendications 1 à 4, caractérisé en ce qu'il consiste à définir au sein du cheminement de faible section et de grande longueur correspondant au parcours des matières traitées, quatre zones successives correspondant chacune à une réaction dominante à savoir :
a) une première zone affectée à une réaction d'hydrolyse et située entre l'entrée principale et une première entrée secondaire,
b) une seconde zone en aval le long du cheminement des matières traitées, et affectée à une réaction d'acidogénèse, située entre la première entrée secondaire et une seconde entrée secondaire ;

c) une troisième zone située à son tour en aval le long du cheminement des matières traitées, et affectée à une réaction d'acétogénèse, cette troisième zone étant située entre la seconde entrée secondaire et une troisième entrée secondaire ; et

d) une quatrième zone située en aval et correspondant à une réaction de méthanogénèse, ladite zone étant située entre la troisième entrée secondaire et la sortie des matières sous forme d'effluents ;

et en ce qu'il consiste à contrôler les conditions d'alimentation, de recyclage, de température, de réinjection de biogaz, au sein de chaque zone, en fonction de la réaction dominante au sein de cette zone.

6. Procédé selon l'une des revendications 1 à 4, caractérisé en ce qu'il est prévu le long du parcours des matières traitées en transit trois zones successives correspondant aux réactions dominantes :

a) une première zone affectée à l'hydrolyse ;

b) une seconde zone affectée aux réactions d'acidogénèse et d'acétogénèse ; et enfin

c) une dernière zone affectée à une réaction de méthanogénèse;

et en ce qu'il consiste à contrôler les conditions d'alimentation, de recyclage, de température, de réinjection de biogaz, au sein de chaque zone en fonction dominante au sein de cette zone.

7. Procédé selon l'une des revendications 1 à 4, caractérisé en ce que l'on définit le long du cheminement des matières en transit, deux zones successives à savoir :

a) une première zone affectée aux réactions d'hydrolyse, d'acidogénèse et d'acétogénèse ; et

b) une seconde zone affectée à la réaction dominante de méthanogénèse;

et en ce qu'il consiste à contrôler les conditions d'alimentation, de recyclage, de température,de réinjection de biogaz au sein de chaque zone en fonction de la réaction dominante au sein de cette zone.

8. Procédé selon l'une des revendications 1 à 7, caractérisé en ce que, pour le contrôle des conditions spécifiques à une réaction dominante, la prise de mesures physiques et/ou chimiques et/ou biologiques se fait notamment par des mesures de pH, de température, de viscosité des matières en transit, de la composition et du débit du biogaz produit, ceci au sein de chaque zone, et permet d'agir ensuite spécifiquement sur les paramètres de conduite des réactions dominantes au sein de chaque zone, en fonction de l'évolution effectivement constatée de la réaction, en optimisant ainsi les conditions de déroulement de la réaction dominante au sein de chaque zone.

9. Procédé selon l'une des revendications 1 à 8, caractérisé en ce que l'on introduit en mélange au sein de la masse des matières en traitement, des métabolites ou réactifs intermédiaires propres à la flore bactérienne spécifique de la réaction dominante se déroulant dans cette zone et choisis dans la famille consistant en :

a) produits riches en glucose introduits dans la zone affectée à l'acidogénèse ;

b) produits riches en acétate introduits dans la zone de méthanogénèse ;

c) oxygène pur ou mélangé (notamment sous forme d'air) introduit dans la zone initiale d'hydrolyse en vue de provoquer une préfermentation aérobie ; et

d) hydrogène pur ou en mélange introduit dans la zone de méthanogénèse, et apte à provoquer la réduction du $CO_2$ et sa transformation en méthane.

10. Installation pour le traitement de matières organiques, à l'état solide ou pâteux et subissant l'action de microorganismes tels que des colonies bactériennes notamment pour la production de biogaz, l'installation étant du type constitué d'une enceinte close formant réacteur, pourvue d'une entrée principale des matières à traiter et d'une sortie des matières digérées sous forme d'effluents, caractérisée en ce que l'enceinte de section constante faible par rapport à son volume et suivant en plan un parcours en forme de segment de couronne, est reliée par une conduite d'entrée (39), une conduite de sortie (40) et des moyens de prélèvement et d'introduction de matières (33, 34, 35, 36, 37, 38) ou de biogaz (70, 71, 72, 73, 74, 75, 76, 77), à une partie centrale dégagée située au centre dudit segment de couronne, constituant une aire technique (23, 32, 32') comprenant des moyens de contrôle et de conduite du traitement dans l'enceinte en ses divers points.

11. Installation selon la revendication 10, caractérisée en ce qu'elle comporte le long de son parcours lesdits moyens de prélèvement de matière (33, 35, 37) depuis le flux de matière en transit au sein de l'installation et lesdits moyens de réintroduction de matière (34, 36, 38) vers le flux desdites matières en transit, ces moyens étant situés sur la paroi concave de l'enceinte de l'installation.

12. Installation selon la revendication 10 ou 11, caractérisée en ce que l'enceinte du réacteur comporte sur sa partie supérieure, et venus de sa paroi formant couverture, des moyens de séparation tels que des cloisons transversales (41) sensiblement verticales et plongeant dans la matière en cours de réaction, ces cloisons étant aptes à diviser l'espace libre au-dessus de la matière pâteuse en transit dans l'enceinte, en définissant des volumes gazeux indépendants les uns des autres, chaque volume étant relié à une unité centrale (32) apte à recueillir séparément les prélèvements effectués dans lesdites phases gazeuses correspondant à chacun des volumes et d'une composition particulière.

13. Installation selon l'une des revendications 10 à 12, caractérisée en ce que chacune des parois transversales isolant les phases gazeuses des différentes zones, est située immédiatement au niveau de chacun des moyens de transfert (33, 34, 35, 36, 37, 38) correspondant à une introduction secondaire, en délimitant ainsi des zones (42, 43, 44, 45) de réaction, chaque volume gazeux de chaque zone étant séparé des volumes gazeux des autres zones, où se produit une réaction biochimique dominante différente.

14. Installation selon l'une des revendications 10 à 13, caractérisée en ce que le réacteur est constitué d'une capacité dont le volume est défini par au moins deux parois latérales (2, 2') inclinées et se rejoignant à la base du réacteur en formant entre elles un espace évasé par le haut, les bords (3, 3') supérieurs des deux parois étant sensiblement parallèles et étant prolongés aux deux extrémités par deux parois transversales, une première paroi recevant l'entrée principale des matières, l'autre paroi transversale recevant la sortie des produits sous forme d'effluents, et en ce que le réacteur comporte une paroi supérieure formant couvercle (15), reposant de façon étanche par ses deux bords latéraux (12, 12') sur les deux bords (3, 3') supérieurs desdites parois latérales inclinées.

15. Installation selon l'une des revendications 10 à 14, caractérisée en ce que le réacteur est constitué d'une part d'une tranchée creusée dans le sol, ladite tranchée ayant une section de forme évasée vers le haut notamment en forme de V ou de U, et les parois de la tranchée étant pourvues d'éléments de surfaçage définissant les parois latérales (8, 8') inclinées du réservoir et assurant l'étanchéité du volume de cette dernière, et d'autre part, en position supérieure, au-dessus de la tranchée, des éléments de couverture (15, 15'), les bords opposés de chaque élément de couverture reposant sur les bords supérieurs desdits éléments de surfaçage en étant solidarisés sur des éléments de repos formant culée (10, 10') et eux-mêmes solidaires des moyens de surfaçage et de revêtement de la tranchée, les éléments de couverture (15, 15') comportant, interposés entre deux éléments adjacents, des moyens d'étanchéité, et les éléments terminaux de couverture comportant à chaque extrémité du réservoir, une paroi transversale (17) munie d'un rebord (18) prévus pour assurer l'étanchéité à chaque extrémité.

16. Installation selon la revendication 10, caractérisée en ce que l'aire technique (23, 32, 32') comporte des moyens de stockage, de préparation, d'introduction et recyclage des matières traitées ou du biogaz, des moyens de circulation et régulation des fluides de transfert thermique, des moyens d'analyse des matières prélevées dans le réacteur, et de mesure des paramètres des matières traitées et du biogaz produit, des moyens de stockage et d'introduction de réactifs ou métabolites neufs, cette aire technique communiquant par des moyens de manipulation et de déplacement de solides et/ou fluides tels que des canalisations radiales, à divers points du réacteur, l'aire technique étant située à égale distance desdits points du réacteur desservi.

17. Installation selon l'une des revendications 10 à 16, caractérisée en ce que le réacteur en forme de couronne comporte plusieurs rampes d'injection (23', 51, 52) disposées le long au moins d'une de ses parois latérales inclinées et propres à distribuer le gaz injecté depuis l'aire technique, ceci de place en place à la base de l'enceinte, les rampes étant alimentées depuis une source de gaz située dans l'aire technique centrale.

18. Installation selon l'une des revendications 10 à 17, caractérisée en ce que l'aire centrale (23, 32, 32') comporte des moyens d'acheminement des matières depuis ladite zone centrale vers chacune des entrées respectivement principales ou secondaires au sein du réacteur, en vue de permettre l'alimentation et le recyclage du réacteur en ses diverses zones (42, 43, 44, 45) et en ce que l'installation comporte encore des moyens d'acheminement (33, 34, 35, 36, 37, 38) de la matière prélevée depuis le réacteur, au niveau de chaque entrée, en vue du recyclage de cette matière réintroduite par l'entrée précédente.

## Claims

1. Method of continuous treatment of organic raw materials in the solid or pasty state through fermentation under the action of colonies of micro-organisms and wherein take place at least two successive phases, each one corresponding to a prevailing biochemical reaction, of the type in which the materials to be processed are introduced into at least one reactor where they are undergoing the action of the micro-organisms such as colonies of bacteria inducing an anaerobic fermentation with the view of biogas production, the material being intended to be continuously conveyed between a main inlet and an outlet located at both ends of the reactor, the materials being conveyed according to a hydraulic retention time corresponding to the time of residence required for the average useful treatment of the materials included in the feeding, the method being characterized in that it consists :
   - in circulating the material continuously in the reactor longitudinally and along a non rectilinear, concave and in particular curved path of travel ;
   - in a least picking up at least one flux of material in a suitable portion of the travel for reintroducing it upstream in order to carry out an at least partial recycling of the treated materials specific to a prevailing reaction zone, the point of introduction and the point of picking up of the recycled materials being located substantially at the inlet point and at the outlet point, respectively, of a virtual volume defining the said zone within the reactor ;

   in modulating the recycling rate within the said zone in order to adapt the average time of residence of the material in this zone to the time of reproduction of the colonies of specific micro-organisms of this zone ; and in controlling the conditions specific to a prevailing reaction within each one of the successive zones by regularly proceeding with the taking of physical and/or chemical and/or biological measurements adapted to allow the determination of the state of the material and the conditions of evolution of the prevailing reaction within each zone.

2. Method according to claim 1, characterized in that the recycling of the materials between the outlet from a working zone specific to a prevailing reaction and the inlet of this zone is adjusted to permit to seed the new materials entering the zone with colonies of micro-organisms specific to the prevailing reaction within this zone.

3. Method according to one of claims 1 to 2, characterized in that it furthermore consists in limiting the gaseous atmosphere prevailing above each zone by putting in place a separation means such as a transverse upper wall at the places corresponding to the outlet of a zone of prevailing reaction and to the inlet of a subsequent zone corresponding to another prevailing reaction, the gaseous atmospheres located above each zone being thus isolated from each other.

4. Method according to one of claims 1 to 3, characterized in that it furthermore consists in recycling biogas tapped from the gaseous atmosphere of a zone in the material being treated within an anterior or posterior zone, the biogas acting as a chemical or biochemical agent in the development of the prevailing reaction.

5. Method according to one of claims 1 to 4, characterized in that it consists in defining within small section and of great length travel corresponding to the path of travel of the treated materials, four successive zones each one corresponding to one prevailing reaction, namely :
   a) a first zone assigned to a hydrolysis reaction and located between the main inlet and a first secondary inlet,
   b) a second zone downstream along the travel of the treated materials and assigned to an acetogenesis reaction, located between the first secondary inlet and a second secondary inlet ;
   c) a third zone in turn located downstream along the travel of the treated materials and assigned to an acetogenesis reaction, this third zone being located between the second secondary inlet and a third secondary inlet ; and
   d) a fourth zone located downstream and corresponding to a methanogenesis reaction, the said zone being located between the third secondary inlet and the outlet of the materials as effluents ; and

in that it consists in controlling the conditions of feeding, of recycling, of temperature, of biogas reinjection within each zone as a function of the prevailing reaction within this zone.

6. Method according to one claims 1 to 4, characterized in that there is provided along the path of travel of the treated materials in transit three successive zones corresponding to the prevailing reactions :

a) a first zone assigned to the hydrolysis ;

b) a second zone assigned to the acetogenesis and acetogenesis reactions ; and at last

c) a last zone assigned to a methanogenesis reaction ;

and in that it consists in controlling the conditions of feeding, of recycling, of temperature, of biogas reinjection within each zone as a prevailing function within this zone.

7. Method according to one of claims 1 to 4, characterized in that two successive zones are defined along the travel of the materials in transit, namely :

a) a first zone assigned to hydrolosis, acetogenesis and acetogenesis reactions ; and

b) a second zone assigned to the prevailing methanogenesis reaction ;

and in that it consists in controlling the conditions of feeding, of recycling, of temperature, of biogas reinjection within each zone as a function of the prevailing reaction within this zone.

8. Method according to one of claims 1 to 7, characterized in that for the control of the conditions specific to one prevailing reaction, the taking of physical and/or chemical and/or biological measurements is made in particular through measurements of pH, of temperature, of viscosity of the materials in transit, of the composition and of the flowrate of the produced biogas and this within each zone and allows to then act specifically upon the parameters for conducting the prevailing reactions within each zone as a function of the evolution actually detected of the reaction by thus optimizing the conditions of evolution of the prevailing reaction within each zone.

9. Method according to one of claims 1 to 8, characterized in that one introduces in admixture within the mass of the materials being treated, intermediate metabolites or reagents peculiar to the specific bacterial flora of the prevailing reaction taking place in this zone and selected in the family consisting of :

a) products rich in glucose introduced into the zone assigned to the acetogenesis ;

b) products rich in acetate introduced in the zone of methanogenesis ;

c) oxygen which is pure or mixed (in particular in the form of air) introduced into the initial zone of hydrolysis in order to induce an aerobic prefermentation; and

d) hydrogen which is pure or in a mixture introduced into the zone of methanogenesis and able to induce the reduction of the $CO_2$ and its conversion into methane.

10. Installation for the treatment of organic materials in the solid or pasty state and undergoing the action of micro-organisms such as bacterial colonies in particular for the production of biogas, the installation being of the type consisting of a closed enclosure forming a reactor provided with a main inlet for the materials to be processed and with an outlet of the digested materials as effluents, characterized in that the enclosure with a constant small section with respect to its volume and along in a plan view of a path of travel shaped as a ring segment is connected by an input duct (39), an output duct (40) and means for picking up and introducing materials (33, 34, 35, 36, 37, 38) or biogas (70, 71, 72, 73, 74, 75, 76, 77) to a disengaged central portion located in the centre of the said ring segment, constituting a technical area (23, 32, 32') comprising means for controlling and conducting the treatment within the enclosure at its various points.

11. Installation according to claim 10, characterized in that it comprises along its path of travel the said means for picking up material (33, 35, 37) from the flux of material in transit within the installation and the said means for reintroducing material (34, 36, 38) towards the flux of the said materials in transit, these means being located on the concave wall of the enclosure of the installation.

12. Installation according to claim 10 or 11, characterized in that the enclosure of the reactor comprises on its upper portion and coming from its wall forming a cover, separation means such as substantially vertical transverse partitions (41) plunging into the material being under reaction, these partitions being adapted to divide the free space above the pasty material in transit within the enclosure while defining gaseous volumes independent from each other, each volume being connected to a central unit (32) adapted to separately collect the picking up effected within the said gaseous phases corresponding to each one of the volumes and having a particular composition.

13. Installation according to one of claims 10 to 12, characterized in that each one of the transverse walls insulating the gaseous phases of the different zones is located directly at each one of the transfer means (33, 34, 35, 36, 37, 38) corresponding to a secondary introduction, while thus limiting reaction zones (42, 43, 44, 45), each gaseous volume of each zone being separated from the gaseous volumes of the other zones wherein one different prevailing biochemical reaction occurs.

14. Installation according to one of claims 10 to 13, characterized in that the reactor consists of a capacity the volume of which is defined by at least two inclined sidewalls (2, 2') and meeting together at the base of the reactor while forming therebetween an upwards flared space, the upper edges (3, 3') of both walls being substantially parallel and being extended at both ends by two transverse walls, a first wall receiving the main inlet of the materials, the other transverse wall receiving the outlet of the products as effluents and in that the reactor comprises an upper wall forming a cover (15) resting in a fluid-tight fashion with its two side edges (12, 12') upon both upper edges (3, 3') of the said inclined sidewalls.

15. Installation according to one of claims 10 to 14, characterized in that the reactor consists on the one hand of a tranch digged out in the ground, the said tranch having a section of in particular V- or U-shaped upwards flared section and the walls of the tranch being provided with surfacing elements defining the inclined sidewalls (8, 8') of the tank and insuring the fluid-tightness of the volume of the latter and on the other hand in the upper position above the tranch, of covering elements (15, 15'), the opposite edges of each covering element resting upon the top edges of the said surfacing elements while being made fast to rest elements (10, 10') forming an abutment and themselves being fasten to means for surfacing and lining the tranch, the covering elements (15, 15') comprising, interposed between two adjacent elements, sealing means and the terminal covering elements comprising at each end of the tank a transverse wall (17) provided with a flange (18) provided to ensure the fluid-tightness at each end.

16. Installation according to claim 10, characterized in that the technical area (23, 32, 32') comprises means for storing, preparing, introducing and recycling the treated material or the biogas, means for circulating and regulating the heat transfer fluids, means for analysing the materials taken from the reactor and for measuring the parameters of the treated materials and of the produced biogas, means for storing and introducing new reagents or metabolites, this technical area communicating through means for manipulating and displacing solids and/or fluids such as radial ducts at various points of the reactor, the technical area being located at equal distance from the said points of the operated reactor.

17. Installation according to one of claims 10 to 16, characterized in that the ring-shaped reactor comprises several injection pipe systems (23', 51, 52) disposed along at least one of its inclined sidewalls and adapted to distribute the injected gas from the technical area, and this from place to place at the base of the enclosure, the pipe systems being fed from a gas source located in the central technical area.

18. Installation according to one of claims 10 to 17, chararacterized in that the central area (23, 32, 32') comprises means for conveying the materials from the said central zone towards each one of the main or secondary inlets, respectively, within the reactor with a view to allow the supply and the recycling of the reactor at its various zones (42, 43, 44, 45) and in that the installation further comprises means (33, 34, 35, 36, 37, 38) for the conveyance of the material taken from the reactor, at each inlet with a view to recycle (this material reintroduced through the preceding inlet.

**Patentansprüche**

1. Verfahren zur fortlaufenden Behandlung von organischen Rohstoffen im festen oder breiartigen Zustand durch Gären unter der Wirkung von Kolonien von Mikroorganismen und bei welchem wenigstens zwei aufeinanderfolgende Phasen wirken, von denen jede einer vorherrschenden biochemischen Reaktion entspricht, derjenigen Gattung, bei welcher die zu behandelnden Stoffe in wenigstens einen Reaktor eingeführt werden, wo sie der Wirkung von Mikroorganismen, wie eine Anaerobengärung zum Zweck der Biogaserzeugung veranlassenden Kolonien von Bakterien unterworfen werden, wobei die Stoffe bestimmt sind, sich zwischen einem Haupteintritt und einem Austritt, die an den beiden Enden des Reaktors liegen, fortlaufend zu bewegen, wobei die sich gemäss einer der für die mittlere nutzbare Behandlung der in der zufuhrenthaltenen Stoffe notwendigen Aufenthaltzeit entsprechenden hydraulischen Rückhaltezeit durchbewegen, wobei das Verfahren dadurch gekennzeichnet ist, dass es darin besteht :

den Stoff in dem Reaktor in Längsrichtung und entlang einer nicht geradlinigen, konkaven und insbesondere krummen Laufbahn fortlaufend umzuwälzen ;

einen Stofffluss wenigstens in einem geeigneten Bereich der Laufbahn wenigstens zu entnehmen, um ihn stromaufwärts wiedereinzuführen, um eine wenigstens teilweise Rückführung der für einen Bereich mit vorherrschender Reaktion spezifischen behandelten Stoffe durchzuführen, wobei die Eintrittstelle und die Entnahmestelle der rückgeführten Stoffe jeweils im wesentlichen an der Eintrittstelle und an der Austrittstelle eines den besagten Bereich innerhalb des Reaktors bestimmenden virtuellen Volumens liegen ; das Rückführungsverhältnis innerhalb des besagten Bereiches zu modulieren, um die mittlere Aufenthaltzeit der Stoffe in diesem Bereich an die Fortpflanzungszeit der für diesen Bereich spezifischen Kolonien von Mikroorganismen anzupassen ; und

die für eine innerhalb jedem der aufeinanderfolgenden Bereiche vorherrschende Reaktion spezifischen Bedingungen zu kontrollieren, indem man regelmässig den Abgriff von physikalischen und/oder chemischen und/oder biologischen Messungen unternimmt, die geeignet sind, die Bestimmung des Zustandes des Stoffes und die Verhältnisse des Ablaufes der vorherrschenden Reaktion innerhalb jedes Bereiches zu gestatten.

2. Verfahren nach Anspruch 1, dadurch gekennzeichnet, dass die Rückführung der Stoffe zwischen dem Austritt eines für die vorherrschende Reaktion spezifischen Arbeitsbereiches und dem Eintritt dieses Bereiches eingestellt wird, um die Einimpfung der in den Bereich eintretenden neuen Stoffen mit für die vorherrschende Reaktion innerhalb dieses Bereiches spezifischen Kolonien von Mikroorganismen zu ermöglichen.

3. Verfahren nach einem der Ansprüche 1 bis 2, dadurch gekennzeichnet, dass es ausserdem darin besteht, die oberhalb jedes Bereiches herrschenden gasförmigen Atmosphären zu begrenzen, indem man ein Trennmittel wie eine obere Querwand an dem Austritt eines Bereiches mit vorherrschender Reaktion und dem Eintritt eines einer anderen vorherrschenden Reaktion entsprechenden späteren Bereiches entsprechenden Stellen einzusetzen, wobei die oberhalb jedes Bereiches liegenden gasförmigen Atmosphären somit voneinander isoliert sind.

4. Verfahren nach einem der Ansprüche 1 bis 3, dadurch gekennzeichnet, dass es ausserdem darin besteht, der gasförmigen Atmosphäre eines Bereiches entzogenes Biogas in den Stoff in Behandlung innerhalb eines vorgeordneten oder nachgeordneten Bereiches rückzuführen, wobei das Biogas als chemisches oder biochemisches Mittel bei der Entwicklung der vorherrschenden Reaktion wirkt.

5. Verfahren nach einem der Ansprüche 1 bis 4, dadurch gekennzeichnet, dass es darin besteht, innerhalb der der Laufbahn der behandelten Stoffe entsprechenden Laufbahn schwachen Querschnitts und grosser Länge vier aufeinander folgende Bereiche zu bestimmen, von denen jede einer vorherrschenden Reaktion entspricht, und zwar :

a) einen einer hydrolysen Reaktion zugewiesenen und zwischen dem Haupteintritt und einem ersten Sekundäreintritt liegenden ersten Bereich ;

b) einen stromabwärts entlang der Laufbahn liegenden und einer säureerzeugenden Reaktion zugewiesenen, zwischen dem ersten Sekundäreintritt und einem zweiten Sekundäreintritt liegenden zweiten Bereich ;

c) einen seinerseits stromabwärts entlang der Laufbahn der behandelten Stoffe liegenden und einer azetoerzeugenden Reaktion zugewiesenen dritten Bereich, wobei dieser dritte Bereich zwischen dem zweiten Sekundäreintritt und einem dritten Sekundäreintritt liegt ; und

d) einen stromabwärts liegenden und einer methanerzeugenden Reaktion entsprechenden vierten Bereich, wobei der besagte Bereich zwischen dem dritten Sekundäreintritt und dem Austritt der Stoffe als Abgänge liegt ;

und das es darin besteht, die Zufuhr-, Rückführungs-, Temperatur-, Biogaswiedereinführungsverhältnisse innerhalb jedes Bereiches in Abhängigkeit der vorherrschenden Reaktion innerhalb dieses Bereiches zu kontrollieren.

6. Verfahren nach einem der Ansprüche 1 bis 4, dadurch gekennzeichnet, dass entlang der Laufbahn der behandelten sich in Durchgang befindenden Stoffe drei den vorherrschenden Reaktionen entsprechende aufeinander folgende Bereiche vorgesehen sind :

   a) ein der Hydrolyse zugewiesener erster Bereich ;

   b) ein den säureerzeugenden und aceterzeugenden Reaktionen zugewiesener zweiter Bereich ; und schliesslich

   c) ein einer methanerzeugenden Reaktion zugewiesener letzter Bereich ;

und dass es darin besteht, die Zufuhr-, Rückführungs-, Temperatur-, Biogaswiedereinspritzungsverhältnisse innerhalb jedes Bereiches in Abhängigkeit der vorherrschenden Reaktion innerhalb dieses Bereiches zu kontrollieren.

7. Verfahren nach einem der Ansprüche 1 bis 4, dadurch gekennzeichnet, dass man entlang der Laufbahn der sich in Durchgang befindenden Stoffe zwei aufeinanderfolgende Bereiche bestimmt, nämlich :

   a) einen den hydrolysen-,säureerzeugenden und aceterzeugenden Reaktionen zugewiesenen ersten Bereich; und

   b) einen der methanerzeugenden vorherrschenden Reaktion zugewiesenen zweiten Bereich ;

und dass es darin besteht, die Zufuhr-Rückführungs-Temperatur- Biogaswiedereinspritzungsverhältnisse innerhalb jedes Bereiches in Abhängigkeit der innerhalb dieses Bereiches vorherrschenden Reaktion zu kontrollieren.

8. Verfahren nach einem der Ansprüche 1 bis 7, dadurch gekennzeichnet, dass zur Kontrolle der für eine vorherrschende Reaktion spezifischen Verhältnisse, der Abgriff von physikalischen und/oder chemischen und/oder biologischen Messungen, insbesondere durch Messungen des pH, der Temperatur, der Zähflüssigkeit der sich in Durchgang befindenden Stoffe, der Zusammensetzung und des Durchsatzes des Biogases geschieht, und dies innerhalb jedes Bereiches und gestattet, dann spezifisch auf die Parameter zur Steuerung der vorherrschenden Reaktionen innerhalb jedes Bereiches in Abhängigkeit der tatsächlich festgestellten Entwicklung der Reaktion einzuwirken, wobei man somit die Bedingungen des Ablaufes der innerhalb jedes Bereiches vorherrschenden Reaktion optimisiert.

9. Verfahren nach einem der Ansprüche 1 bis 8, dadurch gekennzeichnet, dass man als Gemisch innerhalb der Masse der sich in Behandlung befindenden Stoffe Metaboliten und Zwischenreagenzien, die der für die sich in diesem Bereich ablaufende vorherrschende Reaktion spezifischen bakteriellen Flora eigen sind, einführt, welche in der Familie gewählt werden, die besteht aus :

   a) an Glycose reichen, in den der Säureerzeugung zugewiesenen Bereich eingeführten Erzeugnissen ;

   b) an Acetat reichen, in den methanerzeugenden Bereich eingeführten Erzeugnissen ;

   c) in den ursprünglichen hydrolysen Bereich zwecks der Veranlassung einer aeroben Vorgärung eingeführten reinen oder (insbesondere als Luft) gemischten Sauerstoff ; und

   d) in den methanerzeugenden Bereich eingeführten reinen oder gemischten Wasserstoff, der fähig ist, die Reduktion des $CO_2$ und dessen Umwandlung in Methan zu veranlassen.

10. Anlage für die Behandlung von organischen Stoffen im festen oder breiartigen Zustand, die der Wirkung von Mikroorganismen wie bakteriellen Kolonien insbesondere für die Erzeugung von Biogas unterworfen werden, wobei die Anlage der aus einem einen Reaktor bildenden, mit einem Haupteintritt der zu behandelnden Stoffe und einem Austritt der als Abgänge die gerärten Stoffe versehenen geschlossenen Gefäss gebildeten Gattung, dadurch gekennzeichnet, dass das Gefäss mit gegenüber seinem Volumen schwachen konstanten Querschnitt entlang einer in Draufsicht ringsegmentförmigen Laufbahn durch eine Eintrittsleitung (39), eine Austrittsleitung (40) und Mittel zur Entnahme und Einführung der Stoffe (33, 34, 35, 36, 37, 38) oder von Biogas (70, 71, 72, 73, 74, 75, 76, 77) mit einem im Mittelpunkt des besagten Ringsegments liegenden, frei liegenden mittleren Teil verbunden ist, der einen Mittel zur Kontrolle und zur Steuerung der Behandlung in dem Gefäss an dessen verschiedenen Stellen aufweisenden technischen Bereich (23, 32) bildet.

11. Anlage nach Anspruch 10, dadurch gekennzeichnet, dass sie entlang ihrer Durchgangsstrecke die besagten Mittel (33, 35, 37) zur Stoffentnahme aus dem Fluss des sich innerhalb der Anlage in Durchgang befindenden Stoffes und die besagten Mittel (34, 36, 38) zur Wiedereinführung von Stoff zu dem Fluss der besagten sich im Durchgang befindenden Stoffe hin aufweist, wobeidiese Mittel an der konkaven Wand des Gefässes der Anlage liegen.

12. Anlage gemäss Anspruch 10 oder 11, dadurch gekennzeichnet, dass das Gefäss des Reaktors an seinem oberen Teil und von seiner eine Abdeckung bildende Wandung kommend, Trennmittel wie im wesentlichen senkrechte Quertrennwände (41), die in dem reagierenden Stoff eintauchen aufweist, wobei diese Trennwände fähig sind, den freien Raum oberhalb des im Gefäss sich in Durchgang befindenden breiartigen Stoffes unter Abgrenzung von voneinander unabhängigen gasförmigen Volumen zu unterteilen, wobei jedes Volumen mit einer mittleren Einheit (32) verbunden ist, die fähig ist, wie in den jeden der Volumen entsprechenden und aus einer besonderen Zusammensetzung bestehenden besagten gasförmigen Phasen durchgeführten Entnahmen getrennt aufzunehmen.

13. Anlage gemäss einem der Ansprüche 10 bis 12, dadurch gekennzeichnet, dass jede der die gasförmigen Phasen der verschiedenen Bereiche isolierenden Trennwände unmittelbar im Bereich jedes der eine Sekundäreinführung entsprechenden Überführungsmittel (33, 34, 35, 36, 37, 38) liegt und somit Reaktionsbereiche (42, 43, 44, 45) abgrenzt, wobei jedes gasförmige Volumen jedes Bereiches von den gasförmigen Volumen der anderen Bereiche, wo eine unterschiedliche vorherrschende biochemische Reaktion stattfindet, getrennt ist.

14. Anlage gemäss einem der Ansprüche 10 bis 13, dadurch gekennzeichnet, dass der Reaktor aus einem Behälter besteht, dessen Rauminhalt durch wenigstens zwei geneigte Seitenwände (2, 2') bestimmt wird, die am Fuss des Reaktors unter Bildung untereinander eines nach oben erweiterten Raumes zusammenlaufen, wobei die oberen Rände (3, 3') der beiden Wände im wesentlichen parallel und an den beiden Enden durch zwei Querwände verlängert sind, wobei eine erste Wand den Haupteintritt der Stoffe aufnimmt, wobei die andere Querwand den Austritt der Erzeugnisse als Abgänge aufnimmt und das der Reaktor eine einen Deckel bildende, abdichtend mit seinen beiden Seitenrändern (12, 12') auf den beiden oberen Rändern (3, 3') der besagten geneigten Seitenwände abdichten aufliegende obere Wand aufweist.

15. Anlage nach einem der Ansprüche 10 bis 14, dadurch gekennzeichnet, dass der Reaktor einerseits aus einem in dem Boden gegrabenen Graben, wobei der besagte Graben an dem Querschnitt mit nach oben insbesondere V- oder U-förmig erweiterten Gestalt hat, und die Wände des Grabens mit die geneigten Seitenwände (8, 8') des Behälters bestimmenden und die Abdichtung des Volumens dieses letzteren gewährleistenden Elementen versehen sind, und andererseits in der oberen Stellung oberhalb des Grabens aus Abdeckelementen (15, 15') besteht, wobei die entgegengesetzten Ränder jedes Abdeckelementes auf den oberen Kanten der besagten Oberflächenelemente aufliegen, wobei sie an ein Widerlager (10, 10') bildenden Auflageelemente fest angebracht sind und selbst mit den die Oberfläche und den Überzug des Grabens bildenden Elementen fest verbunden sind, wobei die Abdeckelemente (15, 15') zwischen zwei benachbarten Elementen zwischengefügte Abdichtungsmittel aufweisen und wobei die Endabdeckelemente an jedem Ende des Behälters eine mit einer zur Gewährleistung der Abdichtung an jedem Ende vorgesehene Randleiste (18) versehene Querwand (17) aufweisen.

16. Anlage gemäss Anspruch 10, dadurch gekennzeichnet, dass der technische Bereich (23, 32, 32') Mittel zur Lagerung, zur Vorbereitung, zur Einführung und Rückführung der behandelten Stoffe und des Biogases, Mittel zum Umwälzen und zur Regelung der fliessfähigen Wärmeüberführungsmittel, Mittel zur Analyse der in dem Reaktor entnommenen Stoffe und zur Messung der Parameter der behandelten Stoffe und des erzeugten Biogases, Mittel zur Lagerung und zur Einführung von frischen Reagenzien oder Metaboliten aufweist, wobei dieser technische Bereich durch Mittel zur Handhabung und zur Bewegung von Feststoffen und/oder fliessfähigen Mitteln, wie radiale Leitungen mit verschiedenen Stellen des Reaktors in Verbindung stehen, wobei der technische Bereich in gleicher Entfernung von den besagten bedienten Stellen des Reaktors liegt.

17. Anlage gemäss einem der Ansprüche 10 bis 16, dadurch gekennzeichnet, dass der ringförmige Reaktor mehrere entlang wenigstens einer seiner geneigten Seitenwände angeordneten und zur Verteilung des aus dem technischen Bereich eingespritzen Gases geeignete Einspritzröhren (23', 51, 52) aufweist, wobei die Röhren von einer in dem mittleren technischen Bereich liegenden Gasquelle aus gespeist werden.

18. Anlage nach einem der Ansprüche 10 bis 17, dadurch gekennzeichnet, dass der mittlere Bereich (23, 32, 32') Mittel zur Beförderung der Stoffe von dem besagten Bereich aus zu jedem der jeweiligen Haupt-oder Sekundäreintritten innerhalb des Reaktors hin aufweist, um die Speisung und die Rückführung des Reaktors in seinen verschiedenen Bereichen (42, 43, 44, 45) zu gestatten und dass die Anlage noch Mittel (33, 34, 35, 36, 37, 38) zur Beförderung der aus dem Reaktor im Bereich jedes Eintrittes zum Zweck der Rückführung dieses durch den vorangehenen Eintritt wiedereingeführten Stoffes entnommenen Stoffes aufweist.

Fig.1

Fig.2

*Fig.3*

15'

15

24

2

5

41

I

I

7

6

15"

*Fig.4*

15

12

12'

10'

10

8'

8

Fig. 5a

Fig. 5b